# EUROPEAN PATENT SPECIFICATION

(11) **EP 1 166 926 B1**
(45) Date of publication and mention of the grant of the patent: **17.08.2005**
(21) Application number: 01122088.6
(22) Date of filing: 21.01.1994
(51) Int. Cl.: B22F 3/15, B22F 7/00, B29C 43/00, B29C 43/10, B29C 43/12, B29C 43/14, B30B 11/00, C23C 4/18, A61F 2/30

(54) **Method for forming biocompatible components**
Verfahren zum Formen von biokompatiblen Komponenten
Procédé de formation de composants biocompatibles

(30) Priority: 21.01.1993 US 6747
(43) Date of publication of application: 02.01.2002
(62) Divisional of application: 94907414.0
(73) Proprietor: BIOMET, INC., Warsaw, IN 46580 (US)
(72) Inventor: England, Garry Lee, Warsaw Indiana 46580 (US); Higgins, Joel C., Claypool, Indiana 46510 (US)
(74) Representative: Webb, Andrew John

(56) References cited:
- EP-A- 0 375 469
- US-A- 4 187 210
- US-A- 4 619 792
- US-A- 4 644 942
- US-A- 4 744 943
- US-A- 4 854 496
- US-A- 5 030 402
- US-A- 5 037 928
- US-A- 5 051 218
- US-A- 5 066 454
- US-A- 5 201 766

## Description

The present invention relates generally to biomedical implant devices, and more particularly to a method for reducing voids in a stock of nonfibrous biocompatible material used from the such device.

A natural joint in the human body such as a knee joint may undergo degenerative changes due to a variety of etiologies. When these degenerative changes become advanced and are irreversible, it may ultimately become necessary to replace the natural joint with a prosthetic joint. Such a prosthetic joint often includes several biocompatible components which are formed from high strength synthetic materials. These materials are not only able to accommodate the various loading conditions that the prosthetic joint may encounter, but are also biocompatible with the human body. An example of such high strength synthetic materials is ultra-high molecular weight polyethylene which is often used when there is relative movement between the adjacent metallic surface of a prosthetic joint.

Biocompatible components which are made from ultra-high molecular weight polyethylene are often formed using one of two different techniques. In one technique, a relatively precise amount of polyethylene powder is placed between two halves of a die which are then simultaneously compressed and heated. After the powder is densified using standard sintering techniques, the die is allowed to cool. The biocompatible component is then removed from the die and is sterilized in a manner well-known to those skilled in the art.

In the second technique, a substantially completely consolidated polyethylene stock is first formed and then the biocompatible component is machined from the substantially completely consolidated stock. Several methods exist which may be used to form the substantially completely consolidated stock. In one method, the substantially completely consolidated stock is extruded by placing polyethylene powder in a cylindrical chamber having an opening of a particular shape at one end of the chamber. A hydraulically operated piston located at the other end of the cylinder is then used to compress the polyethylene powder. The force exerted by the piston on the polyethylene powder causes the powder to compact. Heat is also applied to solidify the powder as it moves through the cylinder. In another method for forming a substantially completely consolidated stock, polyethylene powder is placed between two flat plates which are compressed while heat is applied. As this occurs, the polyethylene powder is densified so as to form the substantially completely consolidated stock.

While these two techniques for forming biocompatible components are effective, they nevertheless have certain disadvantages. With respect to the first technique described above, it will be appreciated that only one biocompatible component can be made at one time. Accordingly, this technique is relatively inefficient in terms of the amount of time required to make the biocompatible component. With respect to the second technique in which the biocompatible component is formed from a substantially completely consolidated stock, the resulting consolidated stock may often require a stress relief operation or an annealing operation prior to machining. In addition, when polyethylene stock is formed by heating polyethylene powder between two plates acting under pressure, the resulting may have density gradients or voids due to the relatively nonuniform pressure applied to the powder across the plates.

Methods are known for treating ultra-high molecular weight polyethylene prior to being machined into a biocompatible component. One such method is disclosed in United States Patent No. 5,037,928. However, during the procedure described in this reference, the polyethylene stock is placed under a sufficient pressure so as to induce pressure crystallization of the stock. This pressure crystallization tends to cause increased susceptibility to wear. In addition, the use of this relatively high pressure required that relatively expensive pressure containment vessels be used.

According to the invention there is provided a method of reducing voids in a stock of nonfibrous synthetic biocompatible material as described in claim 1.
FIG. 1 is a sagittal elevational view of a knee joint prosthesis including a biocompatible component in the form of a tibial bearing formed from ultra-high molecular weight polyethylene;
FIG. 2 is a cross-sectional view of a cold isostatic press of the type used in accordance with the teachings of EP-B-0 680 290 from which the present application is divided;
FIG. 3 is a perspective view of the first container used with the cold isostatic press shown in FIG. 2 according to the preferred embodiment of EP-B-0 680 290 from which the present application is divided
FIG. 4 is a cross-sectional view of a hot isostatic press of the type used in accordance with the teachings of the preferred embodiment of the present invention;
FIG. 5 of the second container used in conjunction with the hot isostatic press shown in FIG. 4 according to the preferred embodiment of EP-B-0 680 290 from which the present application is divided;
FIG. 6 is a flow diagram illustrating the steps for forming a biocompatible component according to the preferred embodiment of EP-B-0 680 290 from which the present application is divided;
FIG. 7 is a perspective view of the container used in accordance with the preferred embodiment of the present invention to reduce voids in a stock or biocompatible composite material.

It should be understood that while this invention is described in connection with a particular example thereof, the scope of the invention need not be so limited. Rather, those skilled in the art will appreciate that the following teachings can be used in a much wider variety of applications than the examples specifically mentioned herein.

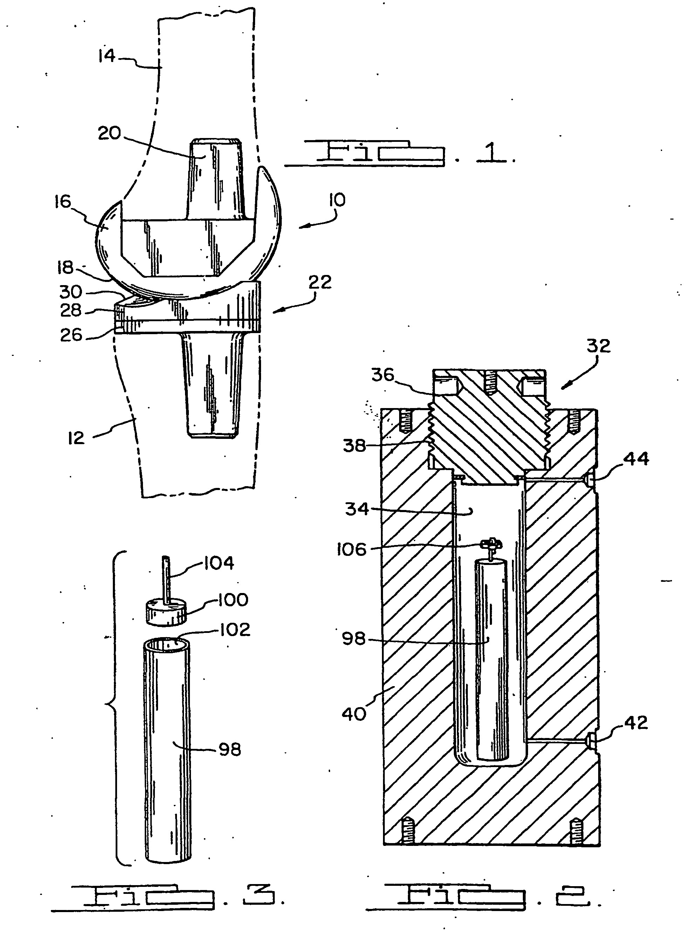

Referring now to FIG. 1, a knee joint prosthesis is shown which is generally designated by the numeral 10. The knee joint prosthesis 10 is functionally depicted as being secured to a tibia 12 and a femur 14 of a surgically resected knee joint, with the tibia 12 and femur 14 being shown in phantom. The knee joint prosthesis 10 is shown to include a femoral component 16 having a bearing surface 18. The femoral component 16 is secured to the femur 14 by means of an inferiorly extending femoral stem 20 inserted into a matching bore created within the femur 14 in a manner well-known to those skilled in the art.

The knee joint prosthesis 10 is further shown to include a tibial component 22 that is secured to the tibia 12 by means of an interiorly extending tibial stem 24 inserted into a matching bore created within the tibia 12 in a similar manner as that described above. The tibial component 22 includes a platform-like tibial tray 26 which is used to support a tibial bearing 28 constructed by the method of the present invention. The tibial bearing 28 is formed to be symmetrically oriented about the sagittal plane. In operation, the tibial bearing 28 provides a bearing surface 30 that is operable to accept a rotatable, low friction contact relationship with the bearing surface 18 of the femoral component 16.

The tibial bearing 28 is formed of a low friction material having enhanced wear resistance properties. The tibial bearing 28 may be machined from a substantially completely consolidated stock that is molded from an ultra-high molecular weight polyethylene powder having a molecular weight of from about 3 million to about 6 million. The ultra-high molecular weight polyethylene powder may be any powder conforming to ASTM F-648, though preferable powders include Hifax 1900 resin available from Himont and GUR 405 or 415 resin available from Hoechst Celanese. It will be understood, however, that other suitable materials may be used to form a stock from which a tibial bearing 28 may be machined. For example, the formation of articles from this method can be accomplished using other polymer materials in powder form, preferably having a molecular weight of from about 3 million to about 6 million. The specific method used to form the tibial bearing 28 includes several steps which are more fully described below. However, several of these steps involve the use of either a cold isostatic press or a hot isostatic press. Accordingly, the structure and operation of the cold isostatic press and the hot isostatic press will now be described.

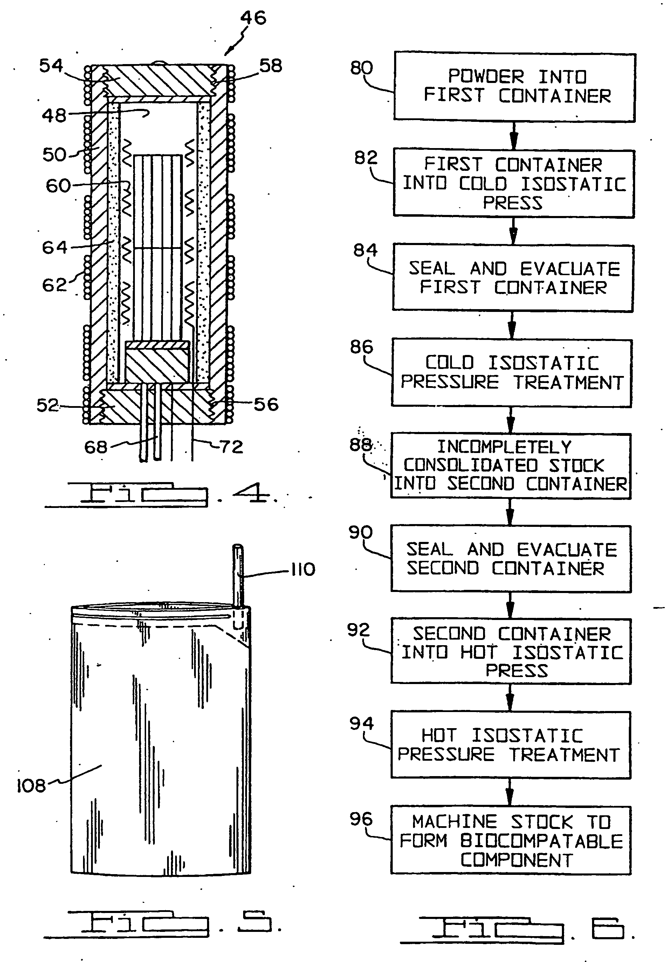

Referring to FIG. 2, a cold isostatic press 32 according to the preferred embodiment of EP-B-0 680 290 from which the present application is divided invention is shown which includes a pressure chamber 34 that has an upper cover 36. The upper cover 36 includes a threaded closure 38 that enhances a sealed condition within the pressure chamber 34 when the pressure chamber 34 is pressurized. When the pressure chamber 34 is sealed in this manner, the length of the pressure chamber 34 is approximately 24-30 inches (61 to 76 cm) and the diameter of the pressure chamber 34 is approximately 12 inches (30 cm). The pressure chamber 34 is substantially surrounded by an annular wall 40 which is operable to define the pressure chamber 34, and has a thickness which is sufficient to contain the pressure within the pressure chamber 34.

The cold isostatic press 32 further includes a pressure inlet line 42 and a pressure relief line 44. The pressure inlet line 42 and pressure relief line 44 are preferably tubular passageways each regulated by a pressure control mechanism (not shown) that are operable to accommodate a pressurized transfer of a gas or liquid fluid from an external source (not shown) into and out of the pressure chamber 34.

The cold isostatic press 32 is preferably designed to operate at pressures capable of compacting the powder to about 60-80% of its desired final density, with the preferable range being between 65-75%. The cold isostatic press 32 may be that which is available from National Forge, Andover, Massachusetts, or Models IP6-24-60 and IP8-36-60 which are available from ABB Autoclave Systems, Inc. of Columbus, Ohio. However, other suitable cold isostatic presses may be used.

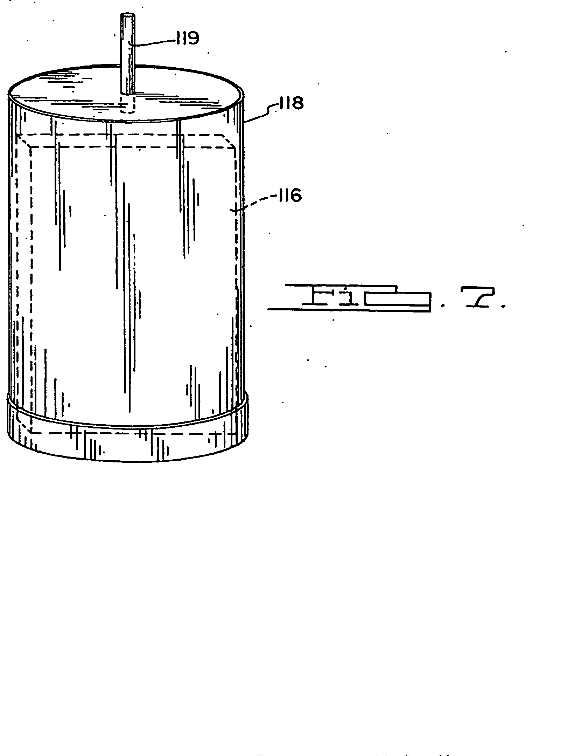

The hot isostatic press 46 will now be described with reference to FIG. 3. The hot isostatic press 46 is shown to include a pressure chamber 48 which is defined in part by an annular wall 50, the thickness of which is between about 6 inches and about 3 inches (15 to 8 cm). In addition, the pressure chamber 48 is about 18 inches (46 cm) in diameter and is about 53 inches (135cm) in length. The hot isostatic press 46 further includes a lower closure 52 and upper closure 54 which are threadedly attached to the annular wall 50 by matching buttress threads 56 and 58. It is to be understood, however, that a pin locking mechanism may also be employed for securing the lower closure 52 and upper closure 54 to the annular wall 50. The lower closure 52 and upper closure 54 are operable to maintain a heated and pressurized condition within the pressure chamber 48 during the hot isostatic pressure treatment described below.

The hot isostatic press 46 further includes a plurality of heating elements 60 that are operable to generate thermal energy within the pressure chamber 48. Alternatively, the hot isostatic press 46 may include another heating means, such as a solution jacket adjacent to the pressure chamber 48, that is operable to contain a hot fluid for providing thermal energy to the pressure chamber 48. The hot isostatic press 46 is also shown to include a cooling jacket 62 which comprises a plurality of coils encircling the annular wall 50. The cooling jacket 62 is operable to contain a suitable heat transfer fluid for removing thermal energy from the hot isostatic press 46 by a transfer of thermal energy into the cooling fluid. It will be understood, however, that the cooling function accomplished by the cooling jacket 62 can be performed by another cooling means disposed at a different location within the hot isostatic press 46, such as within the pressure chamber 48 or between the pressure chamber 48 and the annular wall 50.

The hot isostatic press 46 further includes a heat shield 64 which is located between the annular wall 50 and the heating elements 60. The heat shield 64 is operable to limit heat losses from within the pressure chamber 48 and to assist in controlling the temperature within the pressure chamber 48. The hot isostatic press 46 also includes a pressure system (not shown) of a type well-known to those skilled in the art that is operable to pressurize the pressure chamber 48. The pressure system also communicates with the pressure chamber 48 by means of a pressure input/output line 68 that is connected to an inert gas source and compressor of a type well-known to those skilled in the art. In a preferred embodiment, the inert gas is argon, though nitrogen, helium and neon gases may also be used.

The hot isostatic press 46 further includes a power distribution system (not shown) of a type well-known to those skilled in the art. The power distribution system is used for controlling the heat and pressure within the pressure chamber 48. In addition, the electrical energy required by the heating elements 60 is provided in this embodiment by an electrical power line 72 that is connected to an electrical power source (not shown).

The hot isostatic press 46 is operable to change the temperature within the pressure chamber 48 from an initial room temperature of from about 60° F to about 70° F (16 to 21°C) to an operating temperature of from about 365° F to about 420° F (185 to 216°C). In addition, the hot isostatic press 46 is also operable to change the pressure within the pressure chamber 48 from approximately atmospheric pressure to an operating pressure of preferably from about 7,500 pounds per square inch to about 10,000 pounds per square inch (52 to 69 mPa). The hot isostatic press 46 may be one of several well-known to those skilled in the art, such as Model HP6-30, available from Iso-Spectrum, Inc. of Columbus, Ohio. Other suitable hot isostatic presses are available from National Forge of Andover, Massachusetts. However, other suitable hot isostatic presses may be used.

The method of the preferred embodiment of EP-B-0 680 290 from which the present application is divided will now be described with reference to FIG. 4 which comprises the steps 80 through 96. At step 80, an ultra-high molecular weight polyethylene powder is introduced into a first container 98 (see FIG. 3) so as to substantially fill the first container 98. The first - container 98 is preferably both flexible and collapsible, and is made from a material that has sufficient strength to contain the powder over the operating pressure ranges during the cold isostatic pressure treatment without exhibiting any physical deterioration, chemical degradation or chemical interaction with the powder disposed therein. It is also preferred that the first container 98 be made of a material that will not adhere to the powder at any time during the cold isostatic pressure treatment.

In a preferred embodiment, the first container 98 is a cylindrical polyurethane container of dimensions approximately 6 inches (15cm) in diameter, 18 inches (46 cm) in length, and has a wall thickness of approximately one-half inch to three-fourths inch (1.3 to 1.9 cm). The first container 98 is sealed by means of a plug 100 that is inserted into a matching port 102 at one end of the first container 98. The plug 100 is secured to the first container 98 by means of an adhesive, such as a hot melt glue, located on the top of the interface of the plug 100 and the matching port 102. Because it is desirable that the first container 98 be substantially evacuated prior to the cold isostatic pressure treatment, the plug 100 of the first container 98 preferably includes an evacuation/de-airing tube 104. The evacuation/de-airing tube 104 is operable to be connected to an evacuation pump (not shown) and subsequently sealed by any suitable means prior to the cold isostatic pressure treatment.

It will be noted that the size and shape of the first container 98 will vary depending upon the desired size and shape of the consolidated stock being formed. It will also be noted that other suitable materials may be used to form the first container 98 and that other suitable means may be used for substantially sealing and evacuating the first container 98. For example, a flexible and collapsible rubber material may be employed for constructing the first container 98. When constructed of polyurethane, the first container 98 may be reused provided it is not subjected to extended periods of high temperature.

Once the first container 98 has been filled with powder, the first container 98 is sealed in the manner described above. The first container 98 is then substantially evacuated and then the evacuation/de-airing tube 104 is sealed by any suitable means such as by a clamp 106. As is illustrated by the step 82, the first container 98 is then located within the pressure chamber 34 of the cold isostatic press 32. The pressure chamber 34 is substantially sealed at step 84 to enclose the first container 98 by threading the upper cover 36 onto the matching threads 38 disposed upon the annular wall 40.

The first container 98 is then subjected to a cold isostatic pressure treatment as indicated by the step 86 during which a uniform pressure is applied to the first container 98. In this regard, the pressure applied to the first container 98 is developed by introducing a pressurized fluid into the pressure chamber 34. This pressurized fluid may be water, mineral oil or other oils having similar compressive properties, as well as inert gases such as argon, nitrogen, helium and neon. In addition, the pressure chamber 34 may be partially filled with water while the pressurized gas may be used to fill the remainder of the pressure chamber 34. The pressure within the pressure chamber 34 is preferably increased as quickly as possible from approximately atmospheric pressure to a pressure sufficient to form the powder into an incompletely consolidated stock that can be manipulable for further processing without substantial degradation. Suitable maximum pressures range from 1100 psi to 10,000 psi (7,584 to 68,948 kPa) which are generally sufficient to compact the powder to 60-80% of its final density. Below this range the incompletely consolidated stock is structurally unstable and above this range gases may become trapped within the incompletely consolidated stock during evacuation of the first container 98. In a preferred embodiment, the maximum pressure applied to the first container 98 is approximately 1500 psi (10,342 kPa), and the typical length of time for increasing the pressure to this level may be approximately 2 to 5 minutes. However, maximum pressure applied to the first container 98 is dependent upon several factors including the size of the first container 98, the amount of powder within the first container 98, the size of the resulting stock needed to manufacture the tibial bearing 28 and the size of the pressure chamber 34. The pressure is preferably held at the maximum pressure for approximately one minute, though longer times can be used.

After the maximum pressure within the cold isostatic press 32 is maintained for approximately one minute, the pressure is slowly reduced so as to allow the resulting incompletely consolidated stock to relax within the first container 98 without yielding to outward internal pressure which can cause the incompletely consolidated stock to lose integrity. The pressure is preferably released over a period of from approximately 10 to approximately 30 minutes, although longer times can be used.

The cold isostatic press treatment enhances a uniform density within the incompletely consolidated stock and reduces internal stresses from appearing within the material being formed during the subsequent hot isostatic pressure treatment. In addition, the shape of the incompletely consolidated stock is in large part dependent upon the shape of the first container 98. The incompletely consolidated stock resulting from the cold isostatic press treatment is typically compacted to a preferred density of about 70% of its desired final density following the hot isostatic pressure treatment.

After the incompletely consolidated stock has been removed from the first container 98, the incompletely consolidated stock is placed in a second container 108 (see FIG. 5) as indicated by the step 88. The second container 108 is preferably a collapsible container made from a material that has sufficient strength to contain the incompletely consolidated stock over the temperature and pressure ranges encountered in the hot isostatic press treatment without exhibiting any physical deterioration, chemical degradation or chemical interaction with the incompletely consolidated stock. It is also preferred that the second container 108 be made of a material that will not adhere to the incompletely consolidated stock at any time during the hot isostatic pressure treatment. In a preferred embodiment, the second container 108 is a foilized heat sealable bag that has an external surface formed from a layer of an aluminum foil with a polyester vapor barrier, and has an internal surface formed from a heat-sealable, low density polyethylene layer on its internal surface. The second container 108 may typically be approximately 18 inches (45.8cm) in length, approximately 12 inches in width and have a wall thickness of between approximately 2-3 mils (51 to 76 µm). As will be appreciated by those skilled in the art, the second container 108 may be made from other suitable materials as well.

Because it is desirable to have the second container 108 be substantially evacuated prior to the hot isostatic pressure treatment, the second container 108 preferably includes an evacuation tube 110 that is operable to be connected to a vacuum pump (not shown). In this regard, the evacuation tube 110 is placed in the second container 108 and then a heat sealer is used to seal that region of the second container 108 which is not immediately adjacent to the evacuation tube 110. Hot melt glue is then placed around the region of the second container 108 which is adjacent to the evacuation tube 110.

It will be noted that the size and shape of the second container 108 will vary depending upon the desired size and shape of the consolidated stock being formed. It will also be noted that other suitable materials may be used for the second container 108 and that other suitable means may be used for sealing and evacuating the second container 108.

After the incompletely consolidated stock is placed in the second container 108, the second container 108 is evacuated in similar fashion to the evacuation of the first container 98 as indicated by the step 90. A heat sealer is then used to substantially enclose the second container 108 at a region below the evacuation tube 110. The evacuation tube 110 may then be removed from the second container 108.

Once the incompletely consolidated stock is placed within the second container 108 and the second container 108 is sealed and evacuated. The second container 108 is then placed into the pressure chamber 48 of the hot isostatic press 46 as indicated by the step 92 of the present invention. The lower closure 52 and the upper closure 54 of the hot isostatic press 46 are then closed to substantially enclose the second container 108 within the hot isostatic press 46.

At step 94, the incompletely consolidated stock undergoes the hot isostatic pressure treatment. In this regard, the pressure within the pressure chamber 48 is initially raised to approximately 24 psi (105 kPa) while the temperature of the hot isostatic press 46 is raised between 365°F and 420°F (185 and 216°C). Below this range the incompletely consolidate stock does not melt and above this range the polyethylene may degrade. Preferably, the temperature of the hot isostatic press is raised to between 365° - 385°F (185 to 196°C) to minimize the possibility that degradation will occur. Most preferably, the temperature is raised to 365°F (185°C). Once 365°C (185°C) is reached, the temperature of the hot isostatic press 46 is raised as quickly as possible and may typically heat between one to three hours.

When the temperature of the hot isostatic press 46 reaches approximately 365°F (185°C), the pressure within the pressure vessel 46 is also increased over a 1-2 hours period to a pressure preferably between about 7,500 to about 10,000 psi (51,711 to 68,948 kPa). It will be appreciated that the maximum pressure may range from about 3,000 psi to about 40,000 psi (20,654 to 275,790 kPa). However, pressures below 3,000 psi (20,684 kPa) or above 40,000 psi (275,790 kPa) tend to cause consolidation errors to occur or may cause the resulting completely consolidated stock to have an undesirable crystalline structure. The referred maximum pressure between 7,500 psi and 10,000 psi (51,711 and 68,948 kPa) is dependent upon several factors including the size, shape and construction of the second container, the dimensions of the pressure chamber 48 and the desired final diameter of the resulting completely consolidated stock. In addition, the duration of the hot isostatic pressure treatment may also depend on the size of the resulting completely consolidated stock. For example, smaller diameters of the completely consolidated stock (e.g., 1-1/2 inches (3.8 cm)) typically require less time to become fully compacted, while larger diameters of completely consolidated stock, such as 4 inches (10cm), typically require more time to become fully cured. In addition, the use of the lowest satisfactory pressure is desirable as it would tend to prolong equipment life. An inert gas such as argon is preferably used in the hot isostatic press 46 as the pressure medium. Alternative selections for the pressure medium include nitrogen, helium and neon gases, although these gases can be chemically reactive under certain conditions.

Once the temperature and pressure have reached the desired levels, the temperature and pressure of the pressure chamber 48 remains relatively constant for a given dwell time. During this dwell time, the powder is further compressed so as to minimize any compression release that may occur following termination of the application of heat and pressure. Preferred dwell times are dependent upon the desired final diameter of the consolidated stock being produced, and range from approximately 45 minutes to several hours or more. For example, typical desired dwell times may be approximately 45 minutes to approximately 1 hour for a 1 inch (2.5 cm) diameter consolidated stock, approximately 2 hours for a 2-1/2 inch (6.4 cm) diameter consolidated stock, and approximately 5 hours for a 4 inch (10 cm) diameter consolidated stock.

After the second container 108 has been subjected to the desired temperature and pressure for the given dwell time, the hot isostatic press 46 is allowed to cool to room temperature. After the temperature of the hot isostatic press 46 cools to approximately 100°F (38°C), the pressure within the pressure chamber 48 is gradually decreased to approximately atmospheric pressure over a period of time that is dependent upon the desired final diameter of the consolidated stock being produced. In this regard, the pressure for larger diameters of consolidated stock may be reduced more slowly because they may typically have a larger internal compression and larger potential energy that are more likely to release upon removal of pressure. For example, a 1 hour pressure release time is preferred for a 4 inch (10cm) diameter consolidated stock, while a 20 minute pressure release time may be sufficient for a 1-1/2 inch (3.8cm) diameter consolidated stock. After the release time has elapsed, the pressure chamber 48 is then opened and the second container 108 is removed from the pressure chamber 48.

The consolidated stock is removed from the second container 108 and is machined at step 96 under methods well-known to those skilled in the art to produce the desired product, such as the tibial bearing 28, acetabular cup replacement or other biocompatible component. After machining the consolidated stock at step 92 to form the tibial bearing 28, the tibial bearing 28 is then sterilized in a manner well-known to those skilled in the art.

The isostatic press 46 may also be used in accordance with the present invention to reduce the voids in a stock of biocompatible composite material prior to being machined into a biocompatible component. For example, as shown in FIG. 8, a biocompatible material stock 116 is shown as being disposed within a container 118. The biocompatible material stock 116 may be made from polysulfone, poly ether ether ketone (PEEK), or poly aryl ether ketone (PAEK), though other suitable materials may be used. The container 118 may be a stainless steel or a copper container. However, the container may also be a stainless steel heat treat bag of the type which is available from Sentry Company, Foxboro, Massachusetts, other suitable containers may be used.

When used in this manner, the biocompatible material stock 116 is first placed within the container 118 and then the container 118 is filled with zirconium oxide beads. It will be appreciated, however, that zirconium oxide beads do not have to be used if the container 118 is made from a pliable material such as a heat treat bag. The container 118 is then sealed. A vacuum is then drawn on the container 118 through the evacuation tube 119 and then the evacuation tube 119 is then sealed by closing a valve connected to the evacuation tube 119. The container 118, with the biocompatible stock material 116 located therein, is placed in the isostatic press 46 and the temperature and pressure of the isostatic press 46 are raised to such an extent that the voids formed within the biocompatible stock material 116 are reduced. This reduction in voids occurs because of the external pressure applied to the exterior of the container 118. By using the isostatic press 46 in this manner, the resulting biocompatible material has improved consolidation. The temperature to which the hot isostatic press 46 is raised will be substantially that of the glass transition temperature of the resin of the biocompatible stock material 116, while the pressure applied by the hot isostatic press 46 is as high as reasonably possible provided that it is not more than 10,000 psi (68,948 kPa). The temperature will fall within the range of 400 - 440°F (204 to 225°C) while the pressure will be greater than between 5000 psi and 7500 psi (34,474 and 51,711 kPa), and most preferably greater than 7500 psi (51,711 kPa). However, other suitable temperatures and pressures may be used.

### EXAMPLE 1

This is an example of the method of forming a biocompatible component according to EP-B-0 580 290 from which the present application is divided.

Polyethylene powder having a molecular weight of approximately 3 million and conforming to ASTM F 648-84 is introduced into a first container formed from polyurethane of approximately 6 inches (15cm) in diameter, 18 inches (46cm) in length, and of approximately one-half inch (3.8cm) wall thickness. The first container is substantially sealed and substantially evacuated, and is then placed in the cold isostatic press 32. The cold isostatic press is then closed, and the pressure therein is increased by introducing pressurized water into the pressure vessel to approximately 1500 psi (10,342 kPa) over approximately a 2 minute period. The pressure is then maintained within the cold isostatic press for approximately 1 minute, and is then decreased to atmospheric pressure over approximately a 10 minute period. The cold isostatic press is then opened and the first container is removed and opened to reveal a cylindrical incompletely consolidated stock of dimensions 5-1/2 inches (14cm) in diameter and 14 inches (36cm) in length.

The incompletely consolidated stock is then placed in a second container. The second container is formed from an aluminum foil layer with a polyester vapor barrier on its external surface and a heat-sealable, low density polyethylene layer on its internal surface. In addition, the second container is approximately 18 inches (46 cm) in length, 12 inches (30 cm) in width and has a wall thickness of 2-3 mils (50 to 70). The second container is then evacuated and sealed. The second container is then placed in the hot isostatic press and then the hot isostatic press is closed. The temperature of the hot isostatic press is then increased from room temperature to 400°F (204°C) over approximately a 1 hour period. At the same time, pressurized argon gas is introduced into the pressure chamber of the hot isostatic press to increase the internal pressure from atmospheric pressure to 10,000 pounds per square inch (68,948 kPa) over approximately a 3 hour period. After reaching 400°F (204°C) and 10,000 psi (68,948 kPa), the temperature and pressure within the hot isostatic press are maintained for approximately 5 hours. The temperature is then allowed to decrease to 100°F (38°C) over approximately a 4 hour period. At the same time, the pressure within the hot isostatic press is decreased to atmospheric pressure over a 30 minute period. After release of the pressure and cooling to room temperature, the hot isostatic press is opened and the second container is removed. The second container is then opened to reveal a relatively completely consolidated stock which resembles a cylinder having dimensions of 4 inches (10cm) in diameter and 12 inches (30cm) in length. The relatively completely consolidated stock is tested to have a crystallinity of 45%-60%. The consolidated stock is subsequently machined to form a tibial bearing which is then packaged and then exposed 2.5 megarads of radiation from a cobalt source for sterilization.

### EXAMPLE 2- Example of the Invention

A biocompatible material stock in the form of a slab is placed within a stainless steel container which is then formed with zirconium oxide beads. The slab is approximately 1.5 inches (3.8cm) thick and is 10 (25cm) in length and 10 (25cm) in width. After the container is then evacuated and sealed in the manner described above, the container together with the biocompatible material stock is placed in a hot isostatic press. The temperature of the hot isostatic press is then increased from room temperature to approximately 430°F (220°C) over approximately a .75 hour time period. At the same time, pressurized argon gas is introduced into the hot isostatic press to increase the internal pressure from atmospheric to approximately 7500 psi (51,711 kPa) over approximately a one hour time period.

After reaching 430°F (220°C) and 7500 psi (51,711 kPa), the temperature and pressure of the isostatic press are maintained for approximately .75 hours. The temperature is then reduced to room temperature after which the pressure is released. After pressure has been released and the hot isostatic press has been cooled to room temperature, the hot isostatic press is opened and the container is removed. The container is then opened to reveal a slab of biocompatible material stock in which the voids are reduced.

## Claims

1. A method for reducing voids in a stock of nonfibrous biocompatible synthetic material, said method comprising the steps of:
placing said stock of biocompatible material in a flexible container; and
heating said flexible container in a hot isostatic press to between 400 to 440° F (204 to 225° C) under pressure greater than 5,000 psi (34,474 kPa) up to 10,000 psi (68,948 kPa) so as to reduce the voids in said stock of biocompatible material.

2. A method according to claim 1 wherein said flexible container is heated under pressure greater than 7500 psi (51,711 kPa).

3. A method according to claim 1 or claim 2, wherein said step of placing said stock of biocompatible material in a flexible container includes the step of placing said stock of biocompatible material in a flexible container formed from stainless steel.

4. A method according to any one of claims 1 to 3, wherein said step of heating said flexible container includes the step of heating said flexible container to a temperature substantially that of the glass transition temperature of said stock of biocompatible material.

5. A method according to any preceding claim wherein said nonfibrous biocompatible material stock is made of polysulfone, poly ether ether ketone or poly aryl ether ketone.

## Patentansprüche

1. Verfahren zum Reduzieren von Poren in einem Gut aus nichtfaserigem, biokompatiblen synthetischen Material, wobei das Verfahren die Schritte aufweist:
Platzieren des Gutes aus biokompatiblem Material in einen flexiblen Behälter; und
Erwärmen des flexiblen Behälters in einer heißisostatischen Presse auf 400°F bis 440 °F (204 °C bis 225 °C) unter einem Druck, welcher größer als 5000 psi (34474 kPa) und kleiner als 10000 psi (68948 kPa) ist, um die Poren in dem Gut aus biokompatiblem Material zu reduzieren.

2. Verfahren nach Anspruch 1, wobei der flexible Behälter unter einem Druck erwärmt wird, welcher größer als 7500 psi (51711 kPa) ist.

3. Verfahren gemäß Anspruch 1 oder Anspruch 2, wobei der Schritt des Platzierens des Gutes aus biokompatiblem Material in einen flexiblen Behälter den Schritt des Platzierens des Gutes aus biokompatiblem Material in einen flexiblen Behälter aufweist, welcher aus rostfreiem Stahl gebildet ist.

4. Verfahren nach einem der Ansprüche 1 bis 3, wobei der Schritt des Erwärmens des flexiblen Behälters den Schritt des Erwärmens des flexiblen Behälters auf eine Temperatur aufweist, welche im Wesentlichen die Glasübergangstemperatur des Gutes aus biokompatiblem Material ist.

5. Verfahren nach einem der vorhergehenden Ansprüche, wobei das Gut aus nichtfaserigem, biokompatiblen Material aus Polysulfon, Polyetheretherketon oder Polyaryletherketon hergestellt ist.

## Revendications

1. Procédé pour réduire les espaces vides dans une masse de matériau synthétique biocompatible non fibreux, ledit procédé comprenant les étapes consistant à :
placer ladite masse de matériau biocompatible dans un récipient flexible ; et
chauffer ledit récipient flexible dans une presse isostatique à chaud à une température comprise entre 204 et 225°C (400 et 440°F) sous une pression supérieure à 34 474 kPa (5 000 psi) jusqu'à 68 948 kPa (10 000 psi) afin de réduire les espaces vides dans ladite masse de matériau biocompatible.

2. Procédé selon la revendication 1, dans lequel ledit récipient flexible est chauffé sous une pression supérieure à 51 711 kPa (7 500 psi).

3. Procédé selon la revendication 1 ou la revendication 2, dans lequel ladite étape consistant à placer ladite masse de matériau biocompatible dans un récipient flexible comprend l'étape consistant à placer ladite masse de matériau biocompatible dans un récipient flexible formé à partir d'acier inoxydable.

4. Procédé selon l'une quelconque des revendications 1 à 3, dans lequel ladite étape consistant à chauffer ledit récipient flexible comprend l'étape consistant à chauffer ledit récipient flexible à une température qui est sensiblement celle de la température de transition vitreuse de ladite masse de matériau biocompatible.

5. Procédé selon l'une quelconque des revendications précédentes, dans lequel ladite masse de matériau biocompatible non fibreux est fabriquée à partir de polysulfone, de polyétheréthercétone ou de polyaryléthercétone.
